# EUROPÄISCHE PATENTSCHRIFT

(11) **EP 0 975 257 B1**
(45) Veröffentlichungstag und Bekanntmachung des Hinweises auf die Patenterteilung: **26.02.2003**
(21) Anmeldenummer: 98930668.3
(22) Anmeldetag: 16.04.1998
(51) Int. Cl.: A61B 1/04

(54) **ENDOSKOPISCHES SYSTEM**
ENDOSCOPIC SYSTEM
SYSTEME ENDOSCOPIQUE

(30) Priorität: 16.04.1997 DE 19715951
(43) Veröffentlichungstag der Anmeldung: 02.02.2000
(73) Patentinhaber: Karl Storz GmbH & Co. KG, 78532 Tuttlingen (DE)
(72) Erfinder: IRION, Klaus, M., D-78576 Liptingen (DE); WENZLER, Hartmut, D-78665 Frittlingen (DE); SCHWARZ, Peter, D-78532 Tuttlingen (DE)
(74) Vertreter: Münich, Wilhelm, Dr.
(86) Internationale Anmeldenummer: EP9802224
(87) Internationale Veröffentlichungsnummer: WO98046120

(56) Entgegenhaltungen:
- EP-A- 0 639 349
- WO-A-96/25881
- WO-A-97/19440
- US-A- 5 704 897

## Beschreibung

### Technisches Gebiet

Die Erfindung bezieht sich auf ein endoskopisches System gemäß dem Oberbegriff der Patentansprüche 1, 2 bzw. 4.

Bei den gattungsgemäßen endoskopischen Systemen kann es sich um reale Systeme zum Einsatz am menschlichen oder tierischen Körper oder im technischen Bereich, aber auch um Simulations- bzw. Trainingssysteme handeln, mit denen z.B. ein Arzt endoskopische Eingriffe üben bzw. trainieren kann.

### Stand der Technik

Beispielsweise bei der minimalinvasiven Chirurgie (MIC) wird Video-gestützt operiert. Beim Einführen des oder der Endoskope mit zugehörigem endoskopischem Kamerasystem und dem oder den Instrumenten fehlt dem Mediziner zunächst die visuelle Orientierung des bzw. der Instrumente gegenüber dem oder den Endoskopen. Dies gilt zumindest solange, wie die Instrumente nicht im Blickfeld des Endoskops oder der Endoskope zu sehen sind.

Aber auch dann, wenn der Mediziner das Instrument mit dem Endoskop sieht, fehlt ihm aufgrund des lediglich 2-dimensionalen Bildes zunächst die Information über die Orientierung des Instruments bzw. der Lage des distalen Endes des Instruments bezüglich der Fokusebene.

Ähnliches gilt für die bereits angesprochenen endoskopischen Trainings- bzw. Simulationssysteme. Fortgeschrittene Systeme weisen dabei lediglich ein Gehäuse auf, das den Körper des Patienten simuliert. An dem Gehäuse sind Bedienelemente bzw. Handgriffe angebracht, die den proximalen Elementen der Endoskope bzw. den Handgriffen der Instrumente entsprechen. Die Bewegungen, die der Arzt mit den Bedienelementen bzw. Handgriffen ausführt, werden mittel Positionssensoren erfaßt und in einen Rechner eingegeben. Der Rechner stellt auf einem Bildschirm die Bewegung der bei fortgeschrittenen Simulationssystemen real nicht vorhandenen distalen Funktionselemente in einer "scheinbar realen" Umgebung, also beispielsweise der jeweiligen Körperhöhle unter gleichzeitiger Darstellung von Organen und deren Behandlungsvorgang etc. dar. Damit kann auf dem Bildschirm ein realer Operationsablauf in (fast) allen Einzelheiten simuliert werden.

Zur (vermeintlichen) Lösung des vorstehend angesprochenen Problems der fehlenden Information über die Orientierung der Instrumente sind endoskopische Systeme vorgeschlagen worden, bei denen die Position der Instrumente auf einem Monitor dargestellt wird:

Ein endoskopisches System, von dem bei der Formulierung des Oberbegriffs des Patentanspruchs 1 ausgegangen wird, ist aus der US-PS 5,503,320 oder der entsprechenden EP-A-0 639 349 bekannt. Das in dieser Druckschrift beschriebene System weist wenigstens ein Endoskop auf, dessen Objektiv ein Bild des zu diagnostizierenden oder zu operierenden Bereichs erzeugt. Dieses Bild wird von einem Video-Bildaufnehmer aufgenommen und auf einem Monitor dargestellt. Ferner ist wenigstens ein Instrument, wie eine Schere, ein HF-Instrument, ein Clip-Applikator oder dergleichen vorgesehen, mit dem der eigentliche Behandlungs- bzw. Diagnostiziervorgang vorgenommen wird. Zur Bestimmung der Position des oder der Instrumente ist eine Positionssensor-Einrichtung vorgesehen. Zur Darstellung der Position des oder der Instrumente werden auf dem Monitor Symbole dargestellt, die die Position der Instrumente wiedergeben. Unter Position werden hierbei die Koordinaten eines bestimmten "Punktes", in der Regel des distalen Endes des jeweiligen Instruments verstanden.

Ähnliche Systeme sind aus der EP 0 495 351 B1, der EP 0 672 389 A2, der US-PS 5,253,647, der US-PS 5,383,454 sowie der US-PS 5,417,210 bekannt.

Erfindungsgemäß ist erkannt worden, daß es bei dem aus der gattungsbildenden US-PS 5 503 320 bzw. der EP 0 639 349 A2 bekannten System von Nachteil ist, daß lediglich die Position des distalen Endes bzw. Teile davon, nicht jedoch die Orientierung des Instruments im Raum angezeigt wird. Damit ist es für die Bedienungsperson schwierig, auf dem lediglich zweidimensionalen Bild die Orientierung des Instruments festzustellen. Die Orientierung ist jedoch für die Handhabung des Instruments entscheidend:

Beispielsweise bestimmt die Orientierung beim Vorschieben des Instruments den Ort, auf den das Instrument "als nächstes auftrifft."

### Darstellung der Erfindung

Der Erfindung liegt daher die Aufgabe zugrunde, ein endoskopisches System gemäß dem Oberbegriff der Patentansprüche 1, 2 bzw. 4 derart weiterzubilden, daß die Bedienungsperson das oder die Instrumente schneller in das Blickfeld des Endoskops führen kann, und daß sie darüber hinaus Informationen über die Orientierung der Instrumente erhält.

Eine erfindungsgemäße Lösung dieser Aufgabe ist in den nebengeordneten Patentansprüchen angegeben. Weiterbildungen der Erfindung sind Gegenstand der abhängigen Ansprüche.

Erfindungsgemäß wird von einem endoskopischen System ausgegangen, das wenigstens ein Endoskop, dessen Objektiv ein Bild des zu beobachtenden Bereichs erzeugt, (wenigstens) einen Video-Bildaufnehmer, der das Bild des Objektivs des Endoskops aufnimmt, wenigstens ein Instrument, wie eine Schere, ein HF-Instrument, einem (technischen) Manipulator oder dgl., eine Positionssensor-Einrichtung, die die Position des oder der Instrumente erfaßt, und eine Auswerte- und Steuereinheit aufweist, an der das Ausgangssignal des Video-Bildaufnehmers und der Positionssensor-Einrichtung anliegen, und die ein Bild des von dem Endoskopobjektiv abgebildeten Operationsfeldes auf einem Monitor sowie darüberhinaus Symbole darstellt, die die Position der (beispielsweise chirurgischen) Instrumente wiedergeben.

Dieses endoskopische System wird erfindungsgemäß dadurch weitergebildet, daß die Positionssensor-Einrichtung Sensoren aufweist, die nicht nur die Position, sondern auch die Orientierung sowohl des oder der Endoskope als auch der Instrumente erfassen, und daß die dargestellten Symbole die Orientierung des oder der Instrumente sowie gegebenenfalls der weiteren Endoskope relativ zum dargestellten Bild angeben. Bei Endoskopen, deren Blickrichtung einen Winkel ungleich 0° mit der Längsachse des Endoskops einschließt, also sog. Schrägblick-Endoskopen, kann selbstverständlich auch eine Information über deren Orientierung eingeblendet werden. Weiterhin können auch Informationen über die Orientierung und insbesondere die Drehlage der Instrumente dargestellt werden.

Das endoskopische Instrumentarium, bestehend aus wenigstens einem Endoskop mit Videoaufnahmesystem und wenigstens einem Instrument wird also dahingehend erweitert, daß das oder die Instrumente jeweils einen Positionssensor aufweisen, der sowohl die Lage als auch die Richtung bzw. Orientierung des Instruments bzw. des Endoskops erfaßt. Die Ortsinformation und die zusätzliche Lageinformation werden einer Verarbeitungseinheit zugeleitet, die aus den Orts- und Richtungsinformationen einen sogenannten Richtungsmarker berechnet, der im Videobild des Monitors, bevorzugt im Bildrandbereich des endoskopischen Bildes oder in einem (zusätzlichen) elektronischen Rahmen, der insbesondere um die Bildröhre herum angeordnet sein kann, dargestellt wird. Die Einblendung der (Orts- und) Richtungsinformation am Bildrand erfolgt - wie noch beschrieben wird - bevorzugt nur dann, wenn die Instrumente nicht im Blickfeld des Endoskops liegen. Die Richtungsmarkierung am Bildrand des endoskopischen Bildes auf dem Monitor wird nach Lage, Richtung und/oder Entfernung von einem Referenzpunkt, z.B. auf der Sichtachse des Endoskops codiert.

Parallel oder gegebenenfalls auch alternativ kann auch ein akustisches Führungssignal verwendet werden.

Insbesondere kann das von der Auswerte- und Steuereinheit dargestellte Symbol bzw. das akustische Signal die Eintrittsrichtung und den Eintrittsort des jeweiligen Instruments in das aufgenommene Bild angeben. Weiterhin kann erfindungsgemäß die Auswerte- und Steuereinheit ein Symbol darstellen, das angibt, an welchem Ort die Verlängerung der Längsachse eines nicht im Bild befindlichen Instruments den Bildfeldkegel des Endoskops durchstößt. Dies kann insbesondere dadurch erfolgen, daß das von der Auswerte- und Steuereinheit dargestellte Symbol angibt, unter welchem Azimutwinkel und in welcher Richtung die Verlängerung der Längsachse des Instruments den Bildfeldkegel durchstößt.

In besonders praxisnaher Weise erhält die Bedienungsperson Informationen über die Orientierung und den Ort des jeweiligen Instruments dadurch, daß die Auswerteund Steuereinheit eine Ebene konstruiert, die sich in einem vorgebbaren Abstand vor dem distalen Ende des Endoskops befindet, und daß die Auswerte- und Steuereinheit ein weiteres Symbol darstellt, das angibt, an welcher Stelle die Verlängerung der Längsachse des Instruments diese Ebene durchstößt. Damit weiß die Bedienungsperson, ob das Instrument an dem gewünschten Ort zum Eingriff beispielsweise mit dem zu entfernenden Gewebe kommt.

Diese Ebene kann insbesondere die Ebene sein, auf die das Objektiv fokussiert ist, also die Bildebene.

Weiterhin ist es bevorzugt, wenn das von der Auswerteund Steuereinheit dargestellte Symbol den Abstand zwischen dem distalen Ende des jeweiligen Instruments und dem von dem Objektiv aufgenommenen Bild angibt.

Bei einer weiteren Ausgestaltung der Erfindung gibt das Symbol an, ob die Achse des zugeordneten Instruments den Bildfeldkegel des Objektivs schneidet oder nicht.

In allen vorgenannten Fällen ist es ferner von Vorteil, wenn die jeweilige Angabe durch eine unterschiedliche graphische Codierung des Symbols erfolgt. Die graphische Codierung kann beispielsweise in Art eines Balkendiagramms erfolgen.

Insbesondere können dabei die Sensoren auch die Drehlage der Instrumente und/oder des Endoskops erfassen. Dies ist von besonderer Bedeutung bei sog. Schrägblick-Endoskopen. Die Auswerte- und Steuereinheit ermittelt dann aus dem Ausgangssignal des einem Endoskop zugeordneten Sensors die Orientierung der optischen Achse des Endoskopobjektivs. Entsprechendes gilt für Instrumente, die Manipulationen ausführen, die von der jeweiligen Drehlage des Instruments abhängen.

Bei einer Weiterbildung der Erfindung überlagert die Auswerte- und Steuereinheit die Symbole dem Endoskopbild auf dem Monitor. Dabei ist es insbesondere von Vorteil, wenn die Auswerte- und Steuereinheit die Symbole auf dem Monitor außerhalb des eigentlichen Endoskopbildes darstellt. Hierzu kann - wie bereits beschrieben - ein Rahmen vorgesehen sein, der die Anzeigefläche des Monitors umgibt, wobei die Auswerte- und Steuereinheit die Symbole auf diesem Rahmen darstellt.

Der Rahmen kann lichtemittierende Leuchtelemente, wie Miniaturlämpchen oder Leuchtdioden aufweisen.

Bevorzugt ist es in jedem Fall, wenn die Auswerte- und Steuereinheit ein Symbol nur dann darstellt, wenn - wie bereits beschrieben - das zugeordnete Instrument nicht im Endoskopbild zu sehen ist, da dann die Bedienungsperson nicht mit der Darstellung eigentlich überflüssiger Symbole belastet wird.

Bei dem erfindungsgemäß vorgeschlagenen System können die Sensoren proximal oder distal an den Instrumenten bzw. dem oder den Endoskopen angebracht sein. Dabei können das oder die Endoskope bzw. das oder die Instrumente starre Endoskope bzw. Instrumente oder flexible Endoskope bzw. Instrumente sein. Bei flexiblen Endoskopen bzw. Instrumenten ist es bevorzugt, wenn ein Sensor die jeweilige Abwinklung erfaßt.

Das erfindungsgemäße System kann nicht nur eine Anzeige für eine Bedienungsperson generieren, bei einer Weiterbildung kann die Auswerte- und Steuereinheit ein Führungssystem für das oder die Instrumente steuern. Dieses Führungssystem kann passiv oder aktiv mit der Bedienungsperson kooperieren, also der Führungsperson Hinweise zur Instrumentenbedienung bzw. -positionierung geben oder in die Instrumentenbedienung eingreifen.

Das Führungssystem kann u.a. dann, wenn sich das distale Ende des oder der Instrumente außerhalb des Endoskopbildes befindet, lediglich eine Bewegung des distalen Endes dieses Instruments in das Endoskopbild zulassen. Insbesondere kann das Führungssystem eine Betätigung des jeweiligen Instruments nur dann zulassen, wenn sich das distale Ende dieses Instruments in dem Endoskopbild befindet. Hierzu können die Instrumente eine Markierung aufweisen, die die Auswerte- und Steuereinheit mittels des oder der Endoskope optisch erfaßt, und die als Referenzpunkt für das Führungssystem dient.

Als Endoskope können im Prinzip beliebige Endoskope eingesetzt werden, beispielsweise herkömmliche starre oder flexible Endoskope, bei denen Videoeinheiten an dem Okulartrichter angeflanscht sind. Weiterhin können Endoskope eingesetzt werden, bei denen der Bildaufnehmer der Videoeinheit am distalen Ende angeordnet ist. Besonders bevorzugt ist der Einsatz von Stereoendoskopen, die Stereobilder aufnehmen. Bei derartigen Endoskopen kann die Auswerte- und Steuereinheit mittels Triangulation die Position eines bestimmten Punktes im Endoskopbild festlegen und diesen Punkt als Referenzpunkt für das Führungssystem wählen. In diesem Falle ist es bevorzugt, wenn der Monitor ein Stereodisplay ist, auf dem die Symbole dreidimensional dargestellt werden. Diese dreidimensionalen Symbole können auch den Durchstoßpunkt und die Durchstoßrichtung des jeweiligen Instruments angeben.

Zur Vermessung und damit zur quantitativen Auswertung kann weiterhin eine Projektionseinrichtung vorgesehen sein, die ein zweidimensionales Muster auf den zu diagnostizierenden oder zu operierenden Bereich projiziert. Die Auswerte- und Steuereinheit rekonstruiert dann aus dem Bild des zweidimensionalen Musters die Topographie des Bereichs. Dabei kann das Muster ein Netz oder ein Punkte-Raster sein.

### Kurze Beschreibung der Zeichnung

Die Erfindung wird nachstehend ohne Beschränkung des allgemeinen Erfindungsgedankens anhand eines Ausführungsbeispiels unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:

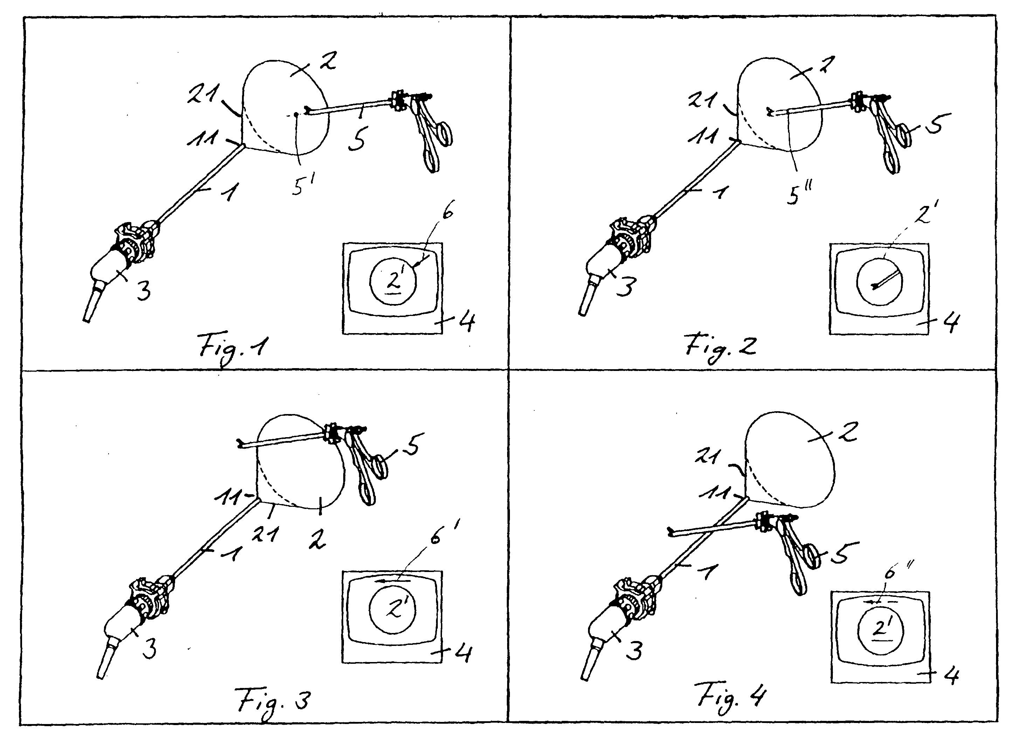

- Fig. 1 bis 4: verschiedene Anordnungen eines Instruments im Bildfeld eines Endoskops und die zugehörigen, auf einem Monitor dargestellten Symbole.

### Darstellung eines Ausführungsbeispiels

In den Fig. 1 bis 4 ist mit 1 ein Endoskop bezeichnet, das bei dem dargestellten Ausführungsbeispiel ohne Beschränkung des allgemeinen Erfindungsgedankens ein starres Endoskop ist. Am distalen Ende 11 des Endoskops 1 ist ein nicht näher dargestelltes Endoskopobjektiv angeordnet. Mit 2 sind die Ebene (Bildebene), auf die das Endoskopobjektiv fokussiert ist, und mit 21 der zugehörige Bildfeldkegel des Endoskopobjektivs bezeichnet.

Bei dem gezeigten Ausführungsbeispiel weist das Endoskop 1 - ohne Beschränkung des allgemeinen Erfindungsgedankens - ein nicht dargestelltes bekanntes - Relaislinsensystem auf, das das von dem Endoskopobjektiv aufgenommene Bild zum proximalen Ende des Endoskops 1 überträgt. Eine an dem Okulartrichter des Endoskops 1 angeflanschte Videoeinheit 3 nimmt das Okularbild auf, so daß es als Bild 2' auf einem Monitor 4 dargestellt werden kann. Ferner ist mit 5 ein Instrument bezeichnet, das zur Ausführung beispielsweise eines diagnostischen oder therapeutischen Vorgangs dient.

Erfindungsgemäß ist eine nicht näher dargestellte Positionssensor-Einrichtung vorgesehen, die Sensoren aufweist, die die Position und die Orientierung des Instruments 5 relativ zur Bildebene 2 sowie zum Bildfeldkegel 21 des Endoskops 1 erfassen. Diese Sensoren können distal oder proximal angeordnet sein. Das Ausgangssignal der Sensoren ist an eine ebenfalls nicht dargestellte Auswerte- und Steuereinheit angelegt, die insbesondere eine Bildverarbeitungseinheit aufweisen kann.

Die Position und die Orientierung des Instruments werden durch Symbole dargestellt, die die nicht näher dargestellte und beispielsweise einen PC bzw. eine Workstation aufweisende Auswerte- und Steuereinheit dem Endoskopbild 2' auf dem Monitor 4 überlagert bzw. neben diesem Bild darstellt.

Bei dem gezeigten Ausführungsbeispiel stellt die Auswerte- und Steuereinheit die Symbole auf dem Monitor 4, jedoch außerhalb des eigentlichen Endoskopbildes 2' dar.

In den Fig. 1 bis 4 sind exemplarisch Symbole (Pfeile 6, 6' und 6") dargestellt, die die Orientierung des In-struments 5 relativ zur Bildebene 2 angeben. Dies soll im folgenden erläutert werden:
Fig. 1 zeigt den Fall, daß das Instrument 5 noch nicht in den Bildfeldkegel 21 des Objektivs eingetreten ist. Die Längsachse des Instruments 5 ist jedoch bereits in einer derartigen Richtung orientiert, daß das Instrument 5 bei einem weiteren Vorschub in den Bildfeldkegel 21 eintritt.
   Dies ist durch einen Pfeil 6 auf dem Monitor 4 symbolisiert, der auf das Bildfeld 2 des Endoskop 1 weist. Mit 5' ist der Durchstoßpunkt der Verlängerung der Längsachse des Instruments 5 durch die Bildebene 2 bezeichnet.
Fig. 2 zeigt den Fall, daß sich das distale Ende des Instruments 5 im Bildfeldkegel 21 befindet. Da die Bedienungsperson das distale Ende des Instruments 5 auf dem Monitor 4 sieht, wird auf die Darstellung eines die Position und die Orientierung des Instruments 5 angebenden Symbols verzichtet, um die Bedienungsperson nicht zu verwirren.
   Allerdings kann - um der Bedienungsperson die dreidimensionale Zuordnung des Instruments 5 zur Bildebene 2 anzugeben - der Durchstoßpunkt 5" des Instruments 5 durch die Bildebene 2 dargestellt werden.
Fig. 3 zeigt den Fall, daß das Instrument 5 sich zwar vor dem distalen Ende 11 des Endoskops 1 befindet, aber derart angeordnet und orientiert ist, daß die Verlängerung der Längsachse des Instruments 5 den Blickfeldkegel 21 nicht schneidet; das Instrument 5 ist vielmehr "über dem Blickfeldkegel 21" angeordnet.
   Diese Anordnung und Orientierung wird durch einen Pfeil 6' symbolisiert, der sich oberhalb des Bildes 2' auf dem Monitor befindet, und dessen Ausrichtung die Lage und Orientierung des Instruments 5 symbolisiert.
Fig. 4 zeigt den Fall, daß sich das Instrument 5 in Blickrichtung vor dem distalen Ende 11 des Endoskops 1 befindet, wobei es so orientiert ist, daß das Instrument bei einem weiteren Vorschub den Blickfeldkegel 21 nicht schneiden kann.
   Diese Orientierung wird bei dem gezeigten Ausführungsbeispiel durch einen unterbrochenen Pfeil 6" symbolisiert.

Vorstehend ist die Erfindung anhand eines Ausführungsbeispiels beschrieben worden. Selbstverständlich sind die verschiedensten Abwandlungen möglich:

So ist es möglich, anstelle eines Endoskops 1 und eines Instruments 5 mehrere Endoskope und/oder Instrumente einzusetzen, deren Lage und Orientierung bezüglich eines oder mehrerer ausgewählten Endoskope und deren Bildfelder auf einem oder mehreren Monitoren dargestellt werden.

Weiterhin ist es möglich, anstelle von oder zusätzlich zu Pfeilen als graphische Symbole Symbole zu verwenden, in denen weitere Informationen codiert sind. Die Codierung dieser Informationen kann beispielsweise in Art von Balkendiagrammen, durch die Farbe der Symbole oder dgl. erfolgen.

Weiterhin können zusätzlich zu den die Lage und die Orientierung angebenden Symbolen, wie Pfeilen oder dgl. weitere, zusätzliche Informationen angebende Symbole dargestellt werden. Die Darstellung dieser Symbole kann überlagert auf dem Endoskopbild 2', außerhalb des Endoskopbildes 2' auf dem Monitor 4 oder außerhalb des eigentlichen Monitors beispielsweise auf dem Umfangsrand des Monitorbildes erfolgen.

Zusätzlich können auch akustische Signale verwendet werden.

Weiterhin können die erfindungsgemäß erhaltenen Positionsinformationen auch als Eingangssignale für Führungssysteme oder dgl. eingesetzt werden.

### Gewerbliche Anwendbarkeit

Das erfindungsgemäße endoskopische System kann im Bereich der Medizin oder der technischen Endoskopie bei allen Arten von endoskopischen Eingriffen, endoskopischen Diagnose- oder Behandlungsvorgängen oder bei endoskopischen Simulations- bzw. Trainingsgeräten eingesetzt werden.

## Patentansprüche

1. Endoskopisches System mit
- wenigstens einem Endoskop (1), dessen Objektiv ein Bild des zu beobachtenden Bereichs erzeugt,
- wenigstens einem Video-Bildaufnehmer (3), der das von dem Objektiv erzeugte Bild aufnimmt,
- wenigstens einem Instrument (5), wie einer Schere, einem HF-Instrument oder einem Manipulator,
- einer Positionssensor-Einrichtung, die die Position des oder der Instrumente (5) bzw. des oder der Endoskope (1) erfaßt, und
- einer Auswerte- und Steuereinheit, an der das Ausgangssignal des Video-Bildaufnehmers (3) und der Positionssensor-Einrichtung anliegen, und die ein Endoskopbild (2') des Bereichs auf einem Monitor (4) und Symbole (6,6',6") darstellt, die die Position des oder der Instrumente (5) wiedergeben,
**dadurch gekennzeichnet, daß** die Positionssensor-Einrichtung Sensoren aufweist, die auch die Orientierung des oder der Instrumente (5) erfassen, daß die Auswerte- und Steuereinheit Symbole (6,6',6") darstellt, die zusätzlich zur Position die Orientierung des oder der Instrumente(5) relativ zur Bildebene (2) des erzeugten Bildes und die Eintrittsrichtung sowie den Eintrittsort des jeweiligen Instruments (5) in das erzeugte Bild angeben.

2. Endoskopisches System mit
- wenigstens einem Endoskop (1), dessen Objektiv ein Bild des zu beobachtenden Bereichs erzeugt,
- wenigstens einem Video-Bildaufnehmer (3), der das von dem Objektiv erzeugte Bild aufnimmt,
- wenigstens einem Instrument (5), wie einer Schere, einem HF-Instrument oder einem Manipulator,
- einer Positionssensor-Einrichtung, die die Position des oder der Instrumente (5) bzw. des oder der Endoskope (1) erfaßt, und
- einer Auswerte- und Steuereinheit, an der das Ausgangssignal des Video-Bildaufnehmers (3) und der Positionssensor-Einrichtung anliegen, und die ein Endoskopbild (2') des Bereichs auf einem Monitor (4) und Symbole (6,6',6") darstellt, die die Position des oder der Instrumente (5) wiedergeben,
**dadurch gekennzeichnet, daß** die Positionssensor-Einrichtung Sensoren aufweist, die auch die Orientierung des oder der Instrumente (5) erfassen, daß die Auswerte- und Steuereinheit Symbole (6,6',6") darstellt, die zusätzlich zur Position die Orientierung des oder der Instrumente(5) relativ zur Bildebene (2) des erzeugten Bildes angeben, sowie weiterhin wenigstens ein Symbol (6,6',6") darstellt, das angibt, an welchem Ort ie Verlängerung der Längsachse eines nicht in der Bildebene (2) befindlichen Instruments (5) den Bildfeldkegel (21) des Endoskops (1) durchstößt.

3. System nach Anspruch 2,
**dadurch gekennzeichnet, daß** das von der Auswerteund Steuereinheit dargestellte Symbol (6,6',6") angibt, unter welchem Azimutwinkel und in welcher Richtung die Verlängerung der Längsachse des Instruments (5) den Bildfeldkegel (21) durchstößt.

4. Endoskopisches System mit
- wenigstens einem Endoskop (1), dessen Objektiv ein Bild des zu beobachtenden Bereichs erzeugt,
- wenigstens einem Video-Bildaufnehmer (3), der das von dem Objektiv erzeugte Bild aufnimmt,
- wenigstens einem Instrument (5), wie einer Schere, einem HF-Instrument oder einem Manipulator,
- einer Positionssensor-Einrichtung, die die Position des oder der Instrumente (5) bzw. des oder der Endoskope (1) erfaßt, und
- einer Auswerte- und Steuereinheit, an der das Ausgangssignal des Video-Bildaufnehmers (3) und der Positionssensor-Einrichtung anliegen, und die ein Endoskopbild (2') des Bereichs auf einem Monitor (4) und Symbole (6,6',6") darstellt, die die Position des oder der Instrumente (5) wiedergeben,
**dadurch gekennzeichnet, daß** die Positionssensor-Einrichtung Sensoren aufweist, die auch die Orientierung des oder der Instrumente (5) erfassen, daß die Auswerte- und Steuereinheit Symbole (6,6',6") darstellt, die zusätzlich zur Position die Orientierung des oder der Instrumente(5) relativ zur Bildebene (2) des erzeugten Bildes angeben, daß die Auswerte- und Steuereinheit eine Ebene konstruiert, die sich in einem vorgebbaren Abstand vor dem distalen Ende des Endoskops (11) befindet, und daß die Auswerte- und Steuereinheit wenigstens ein weiteres Symbol (6,6',6") darstellt, das angibt, an welcher Stelle die Verlängerung der Längsachse des Instruments (5) diese Ebene durchstößt.

5. System nach Anspruch 4,
**dadurch gekennzeichnet, daß** die Bildebene (2) die Ebene ist, auf die das Objektiv fokussiert ist.

6. System nach einem der Ansprüche 1 bis 5,
**dadurch gekennzeichnet, daß** ein von der Auswerteund Steuereinheit dargestelltes Symbol (6,6',6") den Abstand zwischen dem distalen Ende des jeweiligen Instruments(5) und dem von dem Objektiv aufgenommenen Bild angibt.

7. System nach Anspruch 6,
**dadurch gekennzeichnet, daß** das Symbol (6,6',6") in Art einer Leuchtbalkenanzeige ausgebildet ist.

8. System nach einem der Ansprüche 2,3,5 bis 7,
**dadurch gekennzeichnet, daß** eines der Symbole (6,6',6") angibt, ob die Achse des zugeordneten Instruments (5) den Bildfeldkegel (21) des Objektivs schneidet oder nicht.

9. System nach einem der Ansprüche 6 bis 8,
**dadurch gekennzeichnet, daß** die jeweilige Angabe durch eine unterschiedliche graphische Codierung des Symbols (6,6',6") erfolgt.

10. System nach einem der Ansprüche 1 bis 9,
**dadurch gekennzeichnet, daß** die Sensoren auch die Drehlage des oder der Instrumente (5) erfassen.

11. System nach einem der Ansprüche 1 bis 10,
**dadurch gekennzeichnet, daß** die Auswerte- und Steuereinheit aus dem Ausgangssignal eines dem Endoskop (1) zugeordneten Sensors die Orientierung der optischen Achse des Objektivs dieses Endoskops (1) ermittelt.

12. System nach Anspruch 11,
**dadurch gekennzeichnet, daß** die Auswerte- und Steuereinheit die Blickrichtung und den Bildfeldwinkel des jeweils eingesetzten Endoskops (1) berücksichtigt.

13. System nach einem der Ansprüche 1 bis 12,
**dadurch gekennzeichnet, daß** die Auswerte- und Steuereinheit die Symbole (6,6',6") dem auf dem Monitor (4) dargestellten Bild überlagert.

14. System nach Anspruch 13,
**dadurch gekennzeichnet, daß** die Auswerte- und Steuereinheit zumindest einen Teil der Symbole (6,6',6") auf dem Monitor(4) außerhalb des eigentlichen Endoskopbildes darstellt.

15. System nach Anspruch 14,
**dadurch gekennzeichnet, daß** ein Rahmen vorgesehen ist, der die Anzeigefläche des Monitors(4) umgibt, und
daß die Auswerte- und Steuereinheit die Symbole (6,6',6") auf diesem Rahmen darstellt.

16. System nach Anspruch 15,
**dadurch gekennzeichnet, daß** der Rahmen lichtemittierende Leuchtelemente, wie Miniaturlämpchen oder Leuchtdioden aufweist.

17. System nach einem der Ansprüche 2 bis 15 in Verbindung mit Anspruch 2,
**dadurch gekennzeichnet, daß** die Auswerte- und Steuereinheit zumindest die Symbole (6,6',6") für die Eintrittsrichtung und den Eintrittsort in den Bildfeldkegel (21) nur dann darstellt, wenn das zugeordnete Instrument (5) nicht im Endoskopbild zu sehen ist.

18. System nach einem der Ansprüche 4 bis 17 in Verbindung mit Anspruch 4,
**dadurch gekennzeichnet, daß** die Auswerte- und Steuereinheit das Symbol (6,6',6"), das den Durchstoßpunkt durch die Ebene angibt, nur dann darstellt, wenn sich das distale Ende (11) des Instruments (5) in Blickrichtung vor der Ebene befindet, so daß das Instrument (5) die Ebene nicht durchstößt.

19. System nach einem der Ansprüche 1 bis 18,
**dadurch gekennzeichnet, daß** die Sensoren proximal an den Instrumenten (5) bzw. dem Endoskop (1) angebracht sind.

20. System nach Anspruch 19,
**dadurch gekennzeichnet, daß** das Endoskop (1) ein starres Endoskop ist.

21. System nach Anspruch 19,
**dadurch gekennzeichnet, daß** bei einem flexiblen Endoskop (1) oder Instrument (5) zusätzlich ein Sensor vorgesehen ist, der die Abwinkelung des Endoskops (1) oder Instruments (5) erfaßt.

22. System nach einem der Ansprüche 1 bis 18,
**dadurch gekennzeichnet, daß** die Sensoren am distalen Ende (11) der Instrumente (5) bzw. Endoskope (1) angebracht sind.

23. System nach einem der Ansprüche 1 bis 22,
**dadurch gekennzeichnet, daß** die Auswerte- und Steuereinheit ein Führungssystem für das oder die Instrumente (5) steuert.

24. System nach Anspruch 23,
**dadurch gekennzeichnet, daß** das Führungssystem dann, wenn sich das distale Ende des oder der Instrumente außerhalb des Endoskopbildes befindet, lediglich eine Bewegung des distalen Endes (11) dieses Instruments (5) in das Endoskopbild zuläßt.

25. System nach Anspruch 23 oder 24,
**dadurch gekennzeichnet, daß** das Führungssystem eine Betätigung des jeweiligen Instruments (5) nur dann zuläßt, wenn sich das distale Ende (11) dieses Instruments (5) in dem Endoskopbild befindet.

26. System nach einem der Ansprüche 23 bis 25,
**dadurch gekennzeichnet, daß** die Instrumente (5) eine Markierung aufweisen, die die Auswerte- und Steuereinheit mittels des oder der Endoskope (1) optisch erfaßt, und die als Referenzpunkt für das Führungssystem dient.

27. System nach einem der Ansprüche 1 bis 26,
**dadurch gekennzeichnet, daß** das Endoskop (1) ein Stereoendoskop ist.

28. System nach Anspruch 27,
**dadurch gekennzeichnet, daß** die Auswerte- und Steuereinheit mittels Triangulation die Position eines bestimmten Punktes im Endoskopbild festlegt und diesen Punkt als Referenzpunkt für das Führungssystem wählt.

29. System nach Anspruch 27 oder 28,
**dadurch gekennzeichnet, daß** der Monitor (4) ein Stereodisplay ist, auf dem die Symbole (6,6',6") dreidimensional dargestellt werden.

30. System nach Anspruch 29,
**dadurch gekennzeichnet, daß** die dreidimensionalen Symbole (6,6',6") den Durchstoßpunkt (5") und die Durchstoßrichtung des jeweiligen Instruments (5) angeben.

31. System nach einem der Ansprüche 1 bis 30,
**dadurch gekennzeichnet, daß** eine Projektionseinrichtung vorgesehen ist, die ein zweidimensionales Muster auf den zu diagnostizierenden oder zu operierenden Bereich projiziert, und
daß die Auswerte- und Steuereinheit aus dem Bild des zweidimensionalen Musters die Topographie des Bereichs rekonstruiert.

32. System nach Anspruch 31,
**dadurch gekennzeichnet, daß** das Muster ein Netz oder ein Punkte-Raster ist.

33. System nach einem der Ansprüche 1 bis 32,
**dadurch gekennzeichnet, daß** bei bildgebenden Schrägblick-Endoskopen eine Information über den Winkel zwischen der Längsachse des Endoskops (1) und der Blickrichtung dargestellt wird.

34. System nach einem der Ansprüche 1 bis 33,
**dadurch gekennzeichnet, daß** zusätzlich oder alternativ ein akustisches Führungssignal verwendet wird.

## Claims

1. Endoscopic system with
- at least one endoscope (1), the objective lens of which produces an image of an area to be observed;
- at least one video recorder (3) that records the image produced by the objective lens;
- at least one instrument (5) such as cutters, an HF instrument or a manipulator;
- a position sensing means for detecting the position of the instrument or instruments (5) of the endoscope or endoscopes (1); and
- an evaluation and control unit to which the output signal of the video recorder (3) and the position sensing means are connected, and which displays on a monitor (4) an endoscope image (2') of the area, and symbols (6, 6', 6") representing the position of the instrument or instruments (5);
**characterized in that** the position sensing means has sensors that detect also the orientation of the instrument or instruments (5);
that the evaluation and control unit displays symbols (6, 6', 6") indicating, in addition to the position, also the orientation of the instrument or instruments (5) relative to the image plane (2) of the produced image, and the direction of entry as well as the place of entry of a respective instrument (5) into the produced image.

2. Endoscopic system with
- at least one endoscope (1), the objective lens of which produces an image of an area to be observed;
- at least one video recorder (3) that records the image produced by the objective lens;
- at least one instrument (5) such as cutters, an HF instrument or a manipulator;
- a position sensing means for detecting the position of the instrument or instruments (5) of the endoscope or endoscopes (1); and
- an evaluation and control unit to which the output signal of the video recorder (3) and the position sensing means are connected, and which displays on a monitor (4) an endoscope image (2') of the area, and symbols (6, 6', 6") representing the position of the instrument or instruments (5);
**characterized in that** the position sensing means has sensors that detect also the orientation of the instrument or instruments (5);
that the evaluation and control unit displays symbols (6, 6', 6") indicating, in addition to the position, also the orientation of the instrument or instruments (5) relative to the image plane (2) of the produced image, and furthermore also displays at least one symbol (6, 6', 6") indicating the place where the extension of the longitudinal axis of an instrument (5) not located in the image plane (2) penetrates the cone-angle of view (21) of the endoscope (1).

3. System according to claim 2,
**characterized in that** the symbol (6. 6', 6") displayed by the evaluation and control unit indicates the azimuthal angle and the direction of penetration of the cone-angle of view (21) by the extension of the longitudinal axis of the instrument (5).

4. Endoscopic system with
- at least one endoscope (1), the objective lens of which produces an image of an area to be observed;
- at least one video recorder (3) that records the image produced by the objective lens;
- at least one instrument (5) such as cutters, an HF instrument or a manipulator;
- a position sensing means for detecting the position of the instrument or instruments (5) of the endoscope or endoscopes (1); and
- an evaluation and control unit to which the output signal of the video recorder (3) and the position sensing means are connected, and which displays on a monitor (4) an endoscope image (2') of the area, and symbols (6, 6', 6") representing the position of the instrument or instruments (5);
**characterized in that** the position sensing means has sensors that detect also the orientation of the instrument or instruments (5);
that the evaluation and control unit displays symbols (6, 6', 6") indicating, in addition to the position, also the orientation of the instrument or instruments (5) relative to the image plane (2) of the produced image;
that the evaluation and control unit constructs a plane positioned at a predetermined distance in front of the distal end of the endoscope (11); and
that the evaluation and control unit displays at least one further symbol (6, 6', 6") indicating the place at which the extension of the longitudinal axis of the instrument (5) penetrates this plane.

5. System according to claim 4,
**characterized in that** the image plane (2) is the plane onto which the objective lens is focussed.

6. System according to any one of claims 1 to 5,
**characterized in that** a symbol (6, 6', 6") displayed by the evaluation and control unit indicates the distance between the distal end of the respective instrument (5) and the image produced by the objective lens.

7. System according to claim 6,
**characterized in that** the symbol (6, 6', 6") is designed in the manner of a luminous bar display.

8. System according to any one of claims 2, 3, 5 to 7,
**characterized in that** one of the symbols (6, 6', 6") indicates whether or not the axis of the assigned instrument (5) intersects the cone-angle of view (21) of the objective lens.

9. System according to any one of claims 6 to 8,
**characterized in that** the indication concerned is made by means of different graphical coding of the symbol (6, 6', 6").

10. System according to any one of claims 1 to 9,
**characterized in that** the sensors also detect the rotational position of the instrument or instruments (5).

11. System according to any one of claims 1 to 10,
**characterized in that** the evaluation and control unit determines from the output signal of one of the sensors assigned to the endoscope (1) the orientation of the optical axis of the objective lens of this endoscope (1).

12. System according to claim 1,
**characterized in that** the evaluation and control unit takes into account the direction of view and the angle of view of the particular endoscope (1) used.

13. System according to any one of claims 1 to 12,
**characterized in that** the evaluation and control unit superimposes the symbols (6, 6', 6") onto the image displayed on the monitor.

14. System according to claim 13,
**characterized in that** the evaluation and control unit displays the symbols (6, 6', 6") at least partly on the monitor outside the actual endoscope image.

15. System according to claim 14,
**characterized in that** a frame is provided to surround the display surface of the monitor (4); and
that the evaluation and control unit displays the symbols (6, 6', 6") on this frame.

16. System according to claim 15,
**characterized in that** the frame is provided with light emitting members such as miniature lamps or light emitting diodes.

17. System according to any one of claims 2 to 15 in connection with claim 2,
**characterized in that** the evaluation and control unit displays at least the symbols (6, 6', 6") for the direction of entry and the place of entry into the cone-angle of view (21) only when the assigned instrument (5) is not visible in the endoscope image.

18. System according to any one of claims 4 to 17 in connection with claim 4,
**characterized in that** the evaluation and control unit displays the symbol (6, 6', 6") indicating the point of penetration through the plane only when the distal end (11) of the instrument (5) is located in front of the plane in the direction of sight, so that the instrument (5) does not penetrate the plane.

19. System according to any one of claims 1 to 18,
**characterized in that** the sensors are disposed to be proximal to the instruments (5) or the endoscope (1).

20. System according to claim 19,
**characterized in that** the endoscope (1) is a rigid endoscope.

21. System according to claim 19,
**characterized in that**, with a flexible endoscope (1) or instrument (5) a sensor is additionally provided to detect the bending of the endoscope (1 ) or instrument (5).

22. System according to any one of claims 1 to 18,
**characterized in that** the sensors are attached to the distal end (11) of the instruments (5) or endoscopes (1).

23. System according to any one of claims 1 to 22,
**characterized in that** the evaluation and control unit controls a guide system for the instrument or instruments (5).

24. System according to claim 23,
**characterized in that** when the distal end of the instrument or instruments is located outside the endoscope image, the guide system merely permits a movement of the distal end (11) of this instrument (5) into the endoscope image.

25. System according to claim 23 or 24,
**characterized in that** the guide system permits an actuation of the respective instrument (5) only when the distal end (11) of this instrument (5) is located within the endoscope image.

26. System according to any one of claims 23 to 25,
**characterized in that** the instruments (5) are provided with a mark that the evaluation and control unit detects optically by means of the endoscope or endoscopes (1), and that serves as a reference point for the guide system.

27. System according to any one of claims 1 to 26,
**characterized in that** the endoscope (1) is a stereo endoscope.

28. System according to claim 27,
**characterized in that** the evaluation and control unit establishes the position of a particular point in the endoscope image by means of triangulation, and chooses this point as a reference point for the guide system.

29. System according to claim 27 or 28,
**characterized in that** the monitor (4) is a stereo display on that the symbols (6, 6', 6") are displayed three-dimensionally.

30. System according to claim 29,
**characterized in that** the three-dimensional symbols (6, 6', 6") indicate the penetration point (5") and the penetration direction of the respective instrument (5).

31. System according to any one of claims 1 to 30,
**characterized in that** a projection means is provided that projects a two-dimensional pattern onto the area to be diagnosed or operated; and
that the evaluation and control unit reconstructs the topography of the area from the image of the two-dimensional pattern.

32. System according to claim 31,
**characterized in that** the pattern is a grid or a dot matrix.

33. System according to any one of claims 1 to 32,
**characterized in that** with image-forming inclined-view endoscopes information concerning the angle between the longitudinal axis of the endoscope (1) and the direction of viewing is displayed.

34. System according to any one of claims 1 to 33,
**characterized in that** additionally or alternatively an acoustic guide signal is used.

## Revendications

1. Système endoscopique avec
- au moins un endoscope (1), dont l'objectif produit une image de la région à observer,
- au moins un appareil vidéo de prise de vue (3) qui capture l'image produite par l'objectif,
- au moins un instrument (5), tel qu'une paire de ciseaux, un instrument HF ou un manipulateur,
- un dispositif de détection de position, qui détecte la position du ou des instruments (5) ou du ou des endoscopes (1), et
- une unité d'analyse et de commande recevant les signaux de sortie de l'appareil vidéo de prise de vue (3) et du dispositif de détection de position et reproduisant une image endoscopique (2') de la région sur un moniteur (4) ainsi que des symboles (6, 6', 6") qui indiquent la position du ou des instruments,
**caractérisé en ce que** le dispositif de détection de position comporte des détecteurs qui relèvent également l'orientation du ou des instruments (5), **en ce que** l'unité d'analyse et de commande affiche des symboles (6, 6', 6") qui, outre la position, indiquent également l'orientation du ou des instruments (5) par rapport au plan (2) de l'image produite ainsi que la direction d'entrée et le lieu d'entrée de l'instrument (5) dans l'image produite.

2. Système endoscopique avec
- au moins un endoscope (1), dont l'objectif produit une image de la région à observer,
- au moins un appareil vidéo de prise de vue (3) qui capture l'image produite par l'objectif,
- au moins un instrument (5), tel qu'une paire de ciseaux, un instrument HF ou un manipulateur,
- un dispositif de détection de position, qui détecte la position du ou des instruments (5) ou du ou des endoscopes (1), et
- une unité d'analyse et de commande recevant les signaux de sortie de l'appareil vidéo de prise de vue (3) et du dispositif de détection de position et reproduisant une image endoscopique (2') de la région sur un moniteur (4) ainsi que des symboles (6, 6', 6") qui indiquent la position du ou des instruments,
**caractérisé en ce que** le dispositif de détection de position comporte des détecteurs qui relèvent également l'orientation du ou des instruments (5), **en ce que** l'unité d'analyse et de commande affiche des symboles (6, 6', 6") qui, outre la position, indiquent également l'orientation du ou des instruments (5) par rapport au plan (2) de l'image produite, ainsi qu'au moins un symbole (6, 6', 6") indiquant le lieu où le prolongement de l'axe longitudinal d'un instrument (5) non situé dans le plan de l'image (2) traverse l'angle de champ conique (21) de l'endoscope (1).

3. Système selon la revendication 2, **caractérisé en ce que** le symbole (6, 6', 6") affiché par l'unité d'analyse et de commande indique sous quel angle azimutal et dans quelle direction le prolongement de l'axe longitudinal de l'instrument (5) traverse l'angle de champ conique (21).

4. Système endoscopique avec
- au moins un endoscope (1), dont l'object produit une image de la région à observer,
- au moins un appareil vidéo de prise de vue (3) qui capture l'image produite par l'objectif,
- au moins un instrument (5), tel qu'une paire de ciseaux, un instrument HF ou un manipulateur,
- un dispositif de détection de position, qui détecte la position du ou des instruments (5) ou du ou des endoscopes (1), et
- une unité d'analyse et de commande recevant les signaux de sortie de l'appareil vidéo de prise de vue (3) et du dispositif de détection de position et reproduisant une image endoscopique (2') de la région sur un moniteur (4) ainsi que des symboles (6, 6', 6") qui indiquent la position du ou des instruments,
**caractérisé en ce que** le dispositif de détection de position comporte des détecteurs qui relèvent également l'orientation du ou des instruments (5), **en ce que** l'unité d'analyse et de commande affiche des symboles (6, 6', 6") qui, outre la position, indiquent également l'orientation du ou des instruments (5) par rapport au plan (2) de l'image produite, **en ce que** l'unité d'analyse et de commande construit un plan qui se trouve devant et à une distance prédéfinissable de l'extrémité distale de l'endoscope (11), et **en ce que** l'unité d'analyse et de commande affiche au moins un autre symbole (6, 6', 6") indiquant en quel point le prolongement de l'axe longitudinal de l'instrument (5) traverse ce plan.

5. Système selon la revendication 4, **caractérisé en ce que** le plan d'image (2) est le plan sur lequel l'objectif est mis au point.

6. Système selon l'une des revendications 1 à 5, **caractérisé en ce qu'**un symbole (6, 6', 6") affiché par l'unité d'analyse et de commande indique la distance entre l'extrémité distale de l'instrument (5) et l'image produite par l'objectif.

7. Système selon la revendication 6, **caractérisé en ce que** le symbole (6, 6', 6") se présente sous la forme d'un affichage à barres lumineuses.

8. Système selon l'une des revendications 2, 3, 5 à 7, **caractérisé en ce qu'**un des symboles (6, 6', 6") indique si l'axe de l'instrument associé (5) coupe ou non l'angle de champ conique (21) de l'objectif.

9. Système selon l'une des revendications 6 à 8, **caractérisé en ce que** l'indication s'effectue par un codage graphique différent du symbole (6, 6', 6").

10. Système selon l'une des revendications 1 à 9, **caractérisé en ce que** les capteurs relèvent également la position angulaire du ou des instruments (5).

11. Système selon l'une des revendications 1 à 10, **caractérisé en ce que** l'unité d'analyse et de commande détermine, à partir du signal de sortie d'un détecteur associé à l'endoscope (1), l'orientation de l'axe optique de l'objectif de l'endoscope (1) en question.

12. Système selon la revendication 11, **caractérisé en ce que** l'unité d'analyse et de commande prend en compte la direction de visée et l'angle de champ de l'endoscope employé (1).

13. Système selon l'une des revendications 1 à 12, **caractérisé en ce que** l'unité d'analyse et de commande superpose les symboles (6, 6', 6") à l'image représentée sur le moniteur (4).

14. Système selon la revendication 13, **caractérisé en ce que** l'unité d'analyse et de commande affiche au moins une partie des symboles (6, 6', 6") sur le moniteur (4) en dehors de l'image endoscopique proprement dite.

15. Système selon la revendication 14, **caractérisé en ce qu'**il est prévu un cadre entourant la surface d'affichage du moniteur (4) et **en ce que** l'unité d'analyse et de commande affiche les symboles (6, 6', 6") sur ce cadre.

16. Système selon la revendication 15, **caractérisé en ce que** le cadre comporte des éléments produisant de la lumière tels que des lampes miniatures ou des diodes électroluminescentes.

17. Système selon l'une des revendications 2 à 15 en liaison avec la revendication 2, **caractérisé en ce que** l'unité d'analyse et de commande n'affiche au moins les symboles (6, 6', 6") indiquant la direction d'entrée et le lieu d'entrée dans l'angle de champ conique (21) que quand l'instrument associé (5) n'est pas visible sur l'image endoscopique.

18. Système selon l'une des revendications 4 à 17 en liaison avec la revendication 4, **caractérisé en ce que** l'unité d'analyse et de commande n'affiche le symbole (6, 6', 6") indiquant le point de traversée du plan que quand l'extrémité distale (11) de l'instrument (5) se trouve devant le plan dans la direction de visée, de sorte que l'instrument (5) ne traverse pas le plan.

19. Système selon l'une des revendications 1 à 18, **caractérisé en ce que** les détecteurs sont placés à l'extrémité proximale des instruments (5) ou de l'endoscope (1).

20. Système selon la revendication 19, **caractérisé en ce que** l'endoscope (1) est de type rigide.

21. Système selon la revendication 19, **caractérisé en ce qu'**il est également prévu, dans le cas d'un endoscope souple (1) ou d'un instrument souple (5), un détecteur relevant la flexion de l'endoscope (1) ou de l'instrument (5).

22. Système selon l'une des revendications 1 à 18, **caractérisé en ce que** les détecteurs sont rapportés à l'extrémité distale (11) des instruments (5) ou des endoscopes (1).

23. Système selon l'une des revendications 1 à 22, **caractérisé en ce que** l'unité d'analyse et de commande commande un système de guidage du ou des instruments (5).

24. Système selon la revendication 23, **caractérisé en ce que**, quand l'extrémité distale du ou des instruments se trouve à l'extérieur de l'image endoscopique, le système de guidage autorise uniquement un mouvement de l'extrémité distale (11) de l'instrument (5) dans l'image endoscopique.

25. Système selon la revendication 23 ou 24, **caractérisé en ce que** le système de guidage n'autorise l'actionnement de l'instrument (5) que quand l'extrémité distale (11) dudit instrument (5) se trouve dans l'image endoscopique.

26. Système selon l'une des revendications 23 à 25, **caractérisé en ce que** les instruments (5) présentent une marque que l'unité d'analyse et de commande détecte optiquement au moyen du ou des endoscopes (1) et qui sert de point de référence au système de guidage.

27. Système selon l'une des revendications 1 à 26, **caractérisé en ce que** l'endoscope (1) est un endoscope stéréoscopique.

28. Système selon la revendication 27, **caractérisé en ce que** l'unité d'analyse et de commande définit par triangulation la position d'un point donné dans l'image endoscopique et choisit ce point comme point de référence pour le système de guidage.

29. Système selon la revendication 27 ou 28, **caractérisé en ce que** le moniteur (4) est un affichage stéréo sur lequel les symboles (6, 6', 6") sont représentés en trois dimensions.

30. Système selon la revendication 29, **caractérisé en ce que** les symboles tridimensionnels (6, 6', 6") indiquent le point de traversée (5") et la direction de traversée de l'instrument considéré (5).

31. Système selon l'une des revendications 1 à 30, **caractérisé en ce qu'**il est prévu un dispositif de projection qui projète une grille bidimensionnelle sur la région à diagnostiquer ou à opérer, et **en ce que** l'unité d'analyse et de commande reconstruit la topographie de la région à partir de l'image de la grille bidimensionnelle.

32. Système selon la revendication 31, **caractérisé en ce que** la grille est un treillis ou une matrice de points.

33. Système selon l'une des revendications 1 à 32, **caractérisé en ce qu'**est affichée, dans le cas des endoscopes à visée oblique produisant des images, une information sur l'angle formé entre l'axe longitudinal de l'endoscope (1) et la direction de visée.

34. Système selon l'une des revendications 1 à 33, **caractérisé en ce qu'**un signal de guidage acoustique est employé à titre complémentaire ou de variante.
